(19) Europäisches Patentamt
European Patent Office
Office européen des brevets

(11) Publication number : **0 473 337 A2**

(12) # EUROPEAN PATENT APPLICATION

(21) Application number : **91307545.3**

(51) Int. Cl.$^5$ : **A61K 37/64**

(22) Date of filing : **15.08.91**

(30) Priority : **29.08.90 US 574635**

(43) Date of publication of application :
**04.03.92 Bulletin 92/10**

(84) Designated Contracting States :
**AT BE CH DE DK FR GB IT LI LU NL SE**

(71) Applicant : **PFIZER INC.**
**235 East 42nd Street**
**New York, N.Y. 10017 (US)**

(72) Inventor : **LaMattina, John L.**
**13 Huntington Way**
**Ledyard, Connecticut 06339 (US)**

(74) Representative : **Moore, James William, Dr.**
**Pfizer Limited Ramsgate Road**
**Sandwich Kent CT13 9NJ (GB)**

(54) **Nor-statine and nor-cyclostatine polypeptides in the treatment of ocular hypertension and glaucoma.**

(57) The use of nor-statine and nor-cyclostatine polypeptides for the preparation of medicaments for treating glaucoma or ocular hypertension.

EP 0 473 337 A2

The present invention relates to the use of nor-statine and nor-cyclostatine polypeptides for treating patients suffering from glaucoma or ocular hypertension.

Glaucoma is a disease which effects the lives of many people. This eye disorder is caused by an increase in intraocular pressure, also known as ocular hypertension or glaucoma. Chronic glaucoma manifests itself through a gradual and progressive loss of visual fields. Without treatment, such condition ultimately results in blindness. Treatment has typically involved the topical application of agonists or antagonists of autonomic neuroeffectors, such as pilocarpine or timolol, or the systematic administration of carbonic anhydrase inhibitors. Failure of such topical methods leaves surgery as the only alternative. As a result, newer and safer drugs causing fewer side effects are constantly being investigated.

The compounds used in the present invention are known to have a renin inhibitory effect and thus be useful in treating certain forms of hypertension and congestive heart failure as disclosed in United States Patent Nos. 4,814,342 and 4,935,405. W. J. Giardina, et al, Pharmacol., 31, 124 (1989); H. H. Stein, et al, Pharmacol., 31, 124 (1989); and European Patent Publication No. 0364804 refer to the use of certain renin-inhibitory compounds in the treatment of glaucoma.

The present invention relates to the use of an effective intraocular hypertension reducing amount of at least one compound of the formula

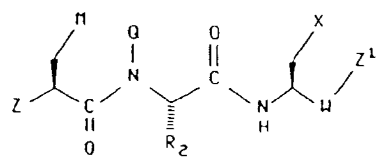

I

and

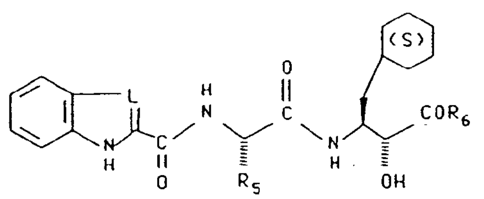

II

or a pharmaceutically acceptable salt thereof, wherein Z is $R_1$- $(Y)_m$- $(A)_p$, where $R_1$ is $(C_1$-$C_6)$alkyl, amino, $(C_1$-$C_4)$alkoxy, $(C_1$-$C_4)$alkylamino, $(C_1$-$C_3)$alkoxy $(C_2$-$C_4)$alkyleneamino, carboxy $(C_1$-$C_4)$alkyl, hydroxy $(C_2$-$C_4)$alkyleneamino, $(C_1$-$C_3)$alkoxy$COCH_2N(CH_3)$, amino $(C_1$-$C_5)$alkyl, morpholino, piperidyl, hydroxypiperidino, 4 -oxopiperidino, piperazino, 4-oxopiperidino ethylene ketal, 4- $(C_1$-$C_3)$ alkylpiperazino, thiomorpholino, thiomorpholino 1-oxide, thiomorpholino 1, 1-dioxide, N- $(C_1$-$C_4)$alkoxycarbonylpiperidyl, 4-$(C_1$-$C_4)$alkoxycarbonylpiperazino, 3 -oxomorpholino, 3, 5-dioxomorpholino, hydroxypyridyl, pyridyl, (s)-pyrrolid-2-yl, N- t-butoxycarbonyl-(s)-pyrrolid-2-yl, $(C_1$-$C_3)$alkoxycarbonyl-(s)-pyrrolid-2-yl or 4-$(C_1$-$C_4)$alkanoylpiperazino; Y is C=O, $P(OCH_3)$ =O or $SO_2$; A is $N(CH_3)$, NH or O; m and p are independently selected from the integers O and 1; M is phenyl, benzyl, naphthyl, thienyl, methoxyphenyl, hydroxyphenyl, chlorophenyl or $(C_6$-$C_7)$cycloalkyl; Q is methyl or hydrogen; $R_2$ is $(C_1$-$C_5)$alkyl, $(C_1$-$C_3)$alkylthio $(C_1$-$C_2)$alkyl, $(C_1$-$C_3)$alkoxy $(C_1$-$C_2)$alkyl, benzyloxy$(C_1$-$C_2)$alkyl, benzyl, hydroxy $(C_1$-$C_2)$alkyl, carboxy$(C_1$-$C_2)$alkyl, guanido$(C_1$-$C_3)$alkyl, $(C_1$-$C_3)$alkylsulfinyl $(C_1$-$C_5)$alkyl, $(C_1$-$C_3)$ alkylsulfonyl $(C_1$-$C_2)$alkyl, 4-benzyloxycarbonylamino-butyl, 4-aminobutyl, imidazol-4-ylmethyl, N-t-butoxycarbonyl imidazol-4 -ylmethyl or carbamyl $(C_1$-$C_2)$alkyl; X is cyclohexyl, i-propyl or phenyl;

W is CH OCO$(C_1$-$C_3)$alkyldi$(C_1$-$C_2)$alkylamino, CH OCO$(C_1$-$C_3)$alkylpiperidino, CH OH, C=O, CH $N_3$, CH $N_3$, CH $NH_2$,

CH $NH_2$, $C(CH_3)$ OH, $C(CH_3)$ OH, CH OCO$(C_1$-$C_2)$alkyl or

CH OCO

$(C_1$-$C_2)$alkylene $CO_2H$; $Z^1$is $CH_2OH$ or R-U-T where R is C=O, U is O, NH, $N(CH_3)$, $CH_2$ or a chemical bond linking R and T; T is $(C_1$-$C_5)$alkyl, hydroxy$(C_1$-$C_4)$alkyl, CONH-$(C_1$-$C_4)$alkyl, hydrogen, trifluoroethyl, $(C_6$-$C_7)$cycloalkyl, $(C_6$-$C_7)$ cycloalkylmethyl, phenyl, benzyl, amino $(C_2$-$C_5)$ alkyl, O- $(C_1$-$C_2)$ alkyl hydroxylamino, mor-

pholino, 4-($C_1$-$C_2$)alkylpiperazino or omega-di($C_1$-$C_2$)alkylamino($C_3$-$C_5$)alkyl; L is CH or N; $R_5$ is imidazol-4-ylmethyl or ($C_2$-$C_5$)alkyl; and $R_5$ is ($C_1$-$C_4$) alkoxy or ($C_1$-$C_4$)alkylamino with the provisos that when m is O, p is O; when A is O, Y is C=O; when T is CONH-($C_1$-$C_4$)alkyl, U is NH, N($CH_3$) or $CH_2$; and when T is ($C_2$-$C_5$)alkylamino, O-($C_1$-$C_2$)alkyl hydroxylamino, morpholino or 4 -($C_1$-$C_2$)-alkylpiperazino, U is $CH_2$ or a chemical bond linking R and T, for the manufacture of medicaments for the treatment of ocular hypertension or glaucoma.

A preferred group of compounds are those of formula I, wherein Y is C=O, A is NH, Q is hydrogen, X is cyclohexyl, W is

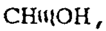 ,

R is C=O, T is benzyl or ($C_1$-$C_5$) alkyl and m and p are each 1. Especially preferred within this group are compounds where $R_1$ is morpholino, M is phenyl, U is O and $R_2$ is n-propyl and T is i-propyl, where $R_2$ is $CH_3SCH_2$- and T is i-propyl, where $R_2$ is n-butyl and T is methyl, where $R_2$ is $HOCH_2$ and T is i-propyl, where $R_2$ is $CH_3O(CH_2)_2$-and T is i-propyl, where $R_2$ is $CH_3SCH_2$- and T is benzyl, where $R_2$ is methyl and T is i-propyl, where $R_2$ is n-butyl and T is i-propyl, where $R_2$ is $CH_3OCH_2$- and T is i-propyl and where $R_2$ is $CH_3CH_22OCH_2$- and T is i-propyl. Also especially preferred within this group are compounds M is phenyl, U is O, T is i-propyl,$R_2$ is $CH_3SCH_2$- and $R_1$ is pyrrolidyl, -pyridyl, piperazino, 4-oxopiperidino or 4-hydroxyypiperidino. Also especially preferred within this group are those compounds where $R_1$ is morpholino, M is phenyl, U is a chemical bond linking R and T and $R_2$ is n-butyl and T is $CH_2CH(CH_3)_2$, and where $R_2$is $CH_3SCH_2$ and T is $CH_2CH(CH_3)_2$. Also especially preferred within this group are compounds where $R_1$ is morpholino, U is O and where M is 2-thienyl, $R_2$ is $CH_3SCH_2$ and T is i-propyl, where M is 4-hydroxyphenyl, $R_2$is $CH_3SCH_2$ and T is i-propyl and where M is 4-methoxyphenyl, $R_2$ is n-butyl and T is methyl. Also especially preferred in this group is a the compound where $R_1$ is morpholino, M is phenyl, $R_2$ is a butyl, U is NH and T is methyl.

A preferrgd group of compounds are those of formula I where M is phenyl, U is a chemical bond linking R and T, T is ($C_1$-$C_5$)alkyl and R is C=O.

The present invention also includes a pharmaceutical composition for use in treating ocular hypertension or glaucoma in an eye, comprising an effective intraocular hypertension reducing amount of at least one compound of formula I and II as defined above, and a pharmaceutically acceptable carrier, said composition being isotonic with tears.

As previously indicated, the present invention utilizes pharmaceutically acceptable salts of the biologically active compounds. Such salts are those which are non-toxic at the dosages administered. Since compounds of the invention may contain basic groups, acid addition salts are possible. Pharmaceutically acceptable acid addition salts include e.g. the hydrochloride, hydrobromide, hydroiodide, sulfate, bisulfate, phosphate, acid phosphate, acetate, lactate, maleate, mesylate, fumarate, citrate, acid citrate, tartrate, bitartrate, succinate, gluconate and saccharate salts.

The compounds of formulae I and II and their pharmaceutically acceptable salts (hereinafter also referred to as active compounds) are described in United States Patent Nos. 4,814,342 and 4,935,405; the disclosure of which is hereby incorporated herein by reference.

The property of reducing intraocular pressure in animals, including man, can be tested using the procedure described by A. M. Tinjum, Acta Opthalmologica, 50, 677 (1972). The procedure involves using New Zealand rabbits weighing 2.0-2.5 kg and placing the rabbits in a restraining device during the testing period. Measurement of intraocular pressure (IOP) is made on the eyes of the rabbits with an applamatic tonometer. For each animal, IOP control measurements are made three times before application of test compounds or vehicle, each time separated by thirty minutes.

After the control measurements have been completed, an amount of test compound or vehicle is applied into the conjunctival sac of one eye of the rabbit. IOP measurements are then taken of that eye and the untreated contralateral eye periodically, e.g. 5, 15, 30, 60, 90, 120, and 150 minutes, after treatment. A reduction in IOP demonstrates intraocular antihypertensive activity.

The present invention is practiced by applying one or more of the nor-statine and nor-cyclostatine compounds topically to the eye in either aqueous solutions, suspensions, or ointments. Typically, these compositions contain approximately 0.001% to 1% of the active compounds and would be applied about twice a day. The amount of the ointment or suspension to be applied to the eye would typically be about 0.1 ml.

The active compounds can be administered using a variety of formulations, including, but not limited to, aqueous solutions, suspensions, and ointments. An aqueous solution for use in the method of the present invention would contain about 0.001% to 1% of active compound in water. The solubility of active compound in the aqueous solution will depend upon the specific active compound used as well as other additives present. A liquid suspension for use in the method of the present invention would contain approximately 0.1 to 10% of active compound in water. Both the aqueous solution and liquid suspension would be buffered to between pH

4.0 and pH 8.0 using, for example, citrate or phosphate buffer. A suitable preservative, for either solution or suspension, such as benzalkium chloride, (about 0.001%) or sorbic acid (about 0.1%) should also be present. Both solution and suspension should be made isotonic with tears using, for example, sodium chloride. A suitable suspending agent would also be necessary for the liquid suspension, such as cellulose (about 0.2% to about 2%), polyvinyl alcohol (about 0. 1% to about 1%), or polyacrylic acid (about 0. 1% to about 0. 5%), preferably cellulose.

An ointment for use in the method of the present invention would contain 0.1 to 10% of active compound. Active compound is suspended in an inert oil-based vehicle, such as, for example, mineral oil or petroleum oil. The ointment should also contain suitable preservatives, such as those used in the water suspension illustrated above.

## Claims

1. The use of an effective intraocular hypertension reducing amount of at least one compound of the formula

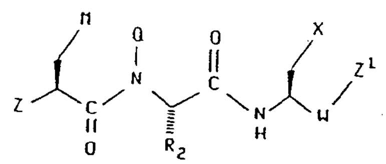

I

and

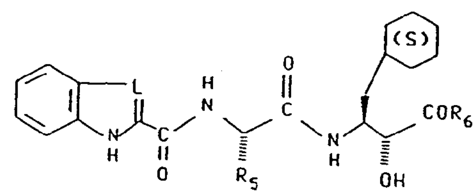

II

or a pharmaceutically acceptable salt thereof, wherein Z is $R_1$-$(Y)_m$-$(A)_p$, where $R_1$ is ($C_1$-$C_6$)alkyl, amino, ($C_1$-$C_4$)alkoxy, ($C_1$-$C_4$)alkylamino, ($C_1$-$C_3$)alkoxy ($C_2$-$C_4$)alkyleneamino, carboxy ($C_1$ -$C_4$)-alkyl, hydroxy($C_2$-$C_4$)-alkyleneamino, ($C_1$-$C_3$)alkoxy$COCH_2N(CH_3)$, amino($C_1$ -$C_5$)-alkyl, morpholino, piperidyl, hydroxypiperidino, 4 -oxopiperidino, piperazino, 4-oxopiperidino ethylene ketal, 4-($C_1$-$C_3$) alkylpiperazino, thiomorpholino, thiomorpholino 1-oxide, thiomorpholino 1, 1 -dioxide, N-($C_1$-$C_4$)alkoxycarbonylpiperidyl, 4-($C_1$-$C_4$)alkoxycarbonylpiperazino, 3-oxomorpholino, 3, 5-dioxomorpholino, hydroxypyridyl, pyridyl, (s)-pyrrolid-2-yl, N-t-butoxycarbonyl-(s)-pyrrolid-2-yl, ($C_1$-$C_3$) alkoxycarbonyl-(s)-pyrrolid-2-yl or 4-($C_1$-$C_4$) alkanoylpiperazino; Y is C=O, $P(OCH_3)$=O or $SO_2$2; A is $N(CH_3)$, NH or O; m and p are independently selected from the integers O and 1; M is phenyl, benzyl, naphthyl, thienyl, methoxyphenyl, hydroxyphenyl, chlorophenyl or ($C_6$-$C_7$) cycloalkyl; Q is methyl or hydrogen; $R_2$ is ($C_1$-$C_5$)alkyl, ($C_1$-$C_3$)alkylthio($C_1$-$C_2$)alkyl, ($C_1$-$C_3$)alkoxy($C_1$-$C_2$)alkyl, benzyloxy($C_1$-$C_2$)alkyl, benzyl, hydroxy ($C_1$-$C_2$)alkyl, carboxy($C_1$-$C_2$)alkyl, guanido($C_1$-$C_3$)alkyl, ($C_1$-$C_3$)alkalkylsulfinyl($C_1$-$C_2$)alkyl, ($C_1$-$C_3$)alkylsulfonyl ($C_1$-$C_2$)alkyl, 4-benzyloxycarbonylaminobutyl, 4-aminobutyl, imidazol-4-ylmethyl, N-t-butoxycarbonylimidazol-4-ylmethyl or carbamyl ($C_1$-$C_2$)alkyl; X is cyclohexyl, i-propyl or phenyl;

W is CH''''''OCO($C_1$-$C_3$)alkyldi($C_1$-$C_2$)alkylamino, CH''''''OCO($C_1$-$C_3$)alkylpiperidino, CH''''''OH, C=O, CH—$N_3$, CH''''''$N_3$, CH—$NH_2$, CH''''''$NH_2$, $C(CH_3)$—OH, $C(CH_3)$''''''OH, CH''''''OCO($C_1$-$C_2$)alkyl or CH''''''OCO($C_1$-$C_2$)alkylene $CO_2H$; $Z^1$ is $CH_2OH$ or R-U-T

where R is C=O, U is O, NH, $N(CH_3)$, $Ch_2$ or chemical bond linking R and T; T is ($C_1$-$C_5$)alkyl, hydroxy ($C_1$-$C_4$)alkyl, CONH-($C_1$-$C_4$)alkyl, hydrogen, trifluoroethyl, ($C_6$-$C_7$)cycloalkyl, ($C_6$-$C_7$)cycloalkylmethyl, phenyl, benyl, amino($C_2$-$C_5$)alkyl, O-($C_1$-$C_2$)alkyl hydroxylamino, morpholino, 4-($C_1$-$C_2$)alkylpiperazino or omega-di($C_1$-$C_2$)alkylamino($C_3$-$C_5$)alkyl; L is CH or N; $R_5$ is imidazol-4-ylmethyl or ($C_2$-$C_5$)alkyl; and $R_6$ is

($C_1$-$C_4$)alkoxy or ($C_1$-$C_4$)alkylamino with the provisos that when m is O, p is O; when A is O, Y is C=O; when T is CONH-($C_1$ -$C_4$)alkyl, U is NH, N($CH_3$) or $CH_2$; and when T is ($C_2$-$C_5$)alkylamino, O-($C_1$-$C_2$)alkyl hydroxylamino, morpholino or 4-($C_1$-$C_2$) alkylpiperazino, U is $CH_2$ or a chemical bond linking R and T, for the manufacture of a medicament for the treatment of ocular hypertension or glaucoma.

**2.** The use, as claimed in claim 1, of a compound of formula 1, wherein Y is C=O, A is NH,Q is hydrogen, X is cyclohexyl, W is

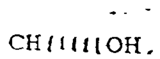

R is C=O, T is benzyl or ($C_1$-$C_5$)alkyl and m and p are each 1.

**3.** The use of a compound as claimed in claim 2, wherein

A. $R_1$ is morpholino, M is 2-thienyl, $R_2$is $CH_3SCH_2$-, U is O and T is i-propyl;
B. $R_1$ is morpholino, M is phenyl, $R_2$is n-propyl, U is O and T is i-propyl;
C. $R_1$is morpholino, M is phenyl, $R_2$is $CH_3SCH_2$-, U is O and T is i-propyl;
D. $R_1$is morpholino, M is phenyl, $R_2$ is n-butyl, U is O and T is methyl;
E. $R_1$ is morpholino, M is phenyl, $R_2$ is n-butyl, U is NH and T is methyl;
F. $R_1$ is morpholino, M is phenyl, $R_2$is n-butyl, U is a chemical bond linking R and T and T is $CH_2CH(CH_3)_2$;
G. $R_1$ is 2-pyrrolidyl, M is phenyl, $R_2$ is $CH_3SCH_2$-, U is O and T is i-propyl;
H. $R_1$ is 4-pyridyl, M is phenyl, $R_2$ is $CH_3SCH_2$-, U is O and T is i-propyl;
I. $R_1$ is,piperazino, M is phenyl, $R_2$ is $CH_3SCH_2$-, U is O and T is i-propyl;
J. $R_1$ is morpholino, M is phenyl, $R_2$ is $CH_3SCH_2$-, U is a chemical bond linking R and T and T is -$CH_2CH(CH_3)_2$;
K. $R_1$ is 4-hydroxypiperidino, M is phenyl, $R_2$ is $CH_3SCH_2$-, U is O and T is i-propyl;
L. $R_1$ is morpholino, M is phenyl, $R_2$ is $HOCH_2$-, U is O and T is i-propyl;
M. $R_1$ is morpholino, M is phenyl, $R_2$ is $CH_3O(CH_2)_2$-, U is O and T is i-propyl;
N. $R_1$ is morpholino, M is phenyl, $R_2$ is $CH_3SCH_2$ -, U is O and T is benzyl;
O. $R_1$ is morpholino, M is 4-hydroxyphenyl, $R_2$ is $CH_3SCH_2$-, U is O and T is i-propyl;
P. $R_1$i s morpholino, M is 4-methoxyphenyl, $R_2$ is n-butyl, U is O and T is methyl;
Q. $R_1$ is morpholino, M is phenyl, $R_2$ is methyl, U is O and T is i-propyl;
R. $R_1$ is 4-oxopiperidino, M is phenyl, $R_2$ is $CH_3SCH_2$-, U is O and T is i-propyl;
S. $R_1$ is morpholino, M is phenyl, $R_2$ is n-butyl, U is O and T is i-propyl;
T. $R_1$ is morpholino, M is phenyl, $R_2$ is $CH_3OCH_2$-, U is O and T is i-propyl; or
U. $R_1$ is morpholino, M is phenyl, $R_2$ is $CH_3CH_2OCH_2$-, U is O and T is i-propyl.

**4.** The use, as claimed in claim 1, of a compound of formula I, wherein M is phenyl, U is a chemical bond linking R and T, T is ($C_1$-$C_5$)alkyl and R is C=0.

**5.** A pharmaceutical composition for topical application for use in treating ocular hypertension or glaucoma in an eye, comprising an effective intraocular hypertension reducing amount of at least one compound of the formula I or II, or a pharmaceutically acceptable salt thereof, as claimed in any one of claims 1 to 4, and a pharmaceutically acceptable carrier, said composition being isotonic with tears.